(19) **Europäisches Patentamt / European Patent Office / Office européen des brevets**

(11) **EP 1 303 240 B1**

(12) **EUROPEAN PATENT SPECIFICATION**

(45) Date of publication and mention
of the grant of the patent:
**13.08.2008  Bulletin 2008/33**

(51) Int Cl.:
***A61F 13/49*** (2006.01)

(21) Application number: **01955868.3**

(22) Date of filing: **19.07.2001**

(86) International application number:
**PCT/US2001/022783**

(87) International publication number:
**WO 2002/007664 (31.01.2002 Gazette 2002/05)**

(54) **METHOD AND APPARATUS UTILIZING SERVO MOTORS FOR PLACING PARTS ONTO A MOVING WEB**

VERFAHREN UND APPARAT, WELCHER SERVOMOTOREN BENUTZT, UM TEILE AUF EINEM SICH BEWEGENDEN GEWEBE ZU PLAZIEREN

PROCEDE ET APPAREIL UTILISANT DES SERVOMOTEURS POUR METTRE EN PLACE DES PIECES SUR UNE BANDE EN DEFILEMENT

(84) Designated Contracting States:
**AT BE CH CY DE DK ES FI FR GB GR IE IT LI LU MC NL PT SE TR**

(30) Priority: **21.07.2000  US 620867**
**29.06.2001  US 897822**

(43) Date of publication of application:
**23.04.2003  Bulletin 2003/17**

(60) Divisional application:
**08103606.3 / 1 941 854**
**08103608.9 / 1 943 994**

(73) Proprietor: **THE PROCTER & GAMBLE COMPANY Cincinnati, Ohio 45202 (US)**

(72) Inventors:
• **BLUMENTHAL, Jeffrey, H.**
**Batavia, OH 45103 (US)**
• **LAMPING, Michael, J.**
**Cincinnati, OH 45252 (US)**

(74) Representative: **McGregor, Judit Ester**
**Procter & Gamble Service GmbH**
**65823 Schwalbach am Taunus (DE)**

(56) References cited:
**EP-A- 0 554 911          EP-A- 0 589 859**
**US-A- 5 766 406          US-A- 6 059 710**

**Description**

FIELD OF THE INVENTION

The present invention relates to a method and apparatus for receiving discrete parts traveling at a speed and applying the parts to a web or other carrier traveling at a different speed.

BACKGROUND OF THE INVENTION

Disposable absorbent articles, such as disposable diapers, generally have been manufactured by a process where discrete parts or components of different materials, such as leg elastic, waist elastic, tapes and other fasteners have been applied to a continuously moving carrier web. Often, the speed at which the parts are fed from one place in the process onto a carrier web is different from the speed of the carrier web, therefore, the speed of the parts must be changed to match the speed of the carrier web to properly apply the parts without adversely affecting the process or the finished product.

Similarly, labels are typically placed onto articles when the speed at which the labels are fed into the process is not the same as the speed of the article to be labeled. Thus, the speed of the labels must be changed to match the speed of the carrier web to properly apply the parts without adversely affecting the process or the finished product.

Several different conventional methods for changing the speed of a part or component of material such that it can be applied to a continuously moving carrier web have been known to those skilled in the art

For example, one method has been known as the slip cut or cut & slip method. A web of material, which is traveling at a slower speed than the carrier web, is fed into a knife and anvil roll having a surface speed equal to speed of the carrier web. The material slips against the surface of the anvil roll until the knife cuts it into discrete pieces. The purpose of the slip is to ensure the correct amount of material is metered into the system at the desired tension prior to cutting. As the material is cut into the discrete parts, vacuum in the anvil roll is activated to hold the discrete part on the anvil without slipping, so that the discrete part is accelerated to the speed of the anvil roll. The anvil roll then carries the part to the point where the vacuum is released and the parts are applied to the carrier web while both the parts and the carrier web are traveling at the same speed. The problem with the above method is that the slip processis very sensitive to material properties and process settings. For example, when the coefficient of friction between the material and anvil roll is too high the material will elongate during the slip process. This elongation, if it occurs, can contribute to high variability in the final cut length and placement of the discrete part on the carrier web.

Another method has used festoons to reduce the speed of the carrier web to match the speed of the discrete parts of material to be applied to the web. An example of this method is described in U.S. Patent No. 5,693,195 issued to Schmitz. The carrier web is temporarily slowed down to the speed of the parts with the excess portion of the carrier web gathering in festoons. The parts of material are then applied to the carrier web while both the parts and the web are traveling at the same speed. The festoons are then released allowing the moving web to return to its original speed. This method has two main drawbacks. First, the carrier web must be festooned and then released; this may damage or otherwise change the properties of the carrier web. Second, the storage system requires a large amount of space in typical disposables production systems because there is a direct relationship between line speed and storage space needed.

Another method has utilized a cam actuated follower arm. The cam actuated follower comprises a cam follower at one end of the arm and a holding plate at the other end of the arm. The cam follower remains in contact with a fixed cam which is mounted concentric with the instantaneous center of rotation of the holding plate. As the holding plate rotates, its radial distance from the censer of rotation is increased and decreased to change the surface speed of the holding plate. The discrete parts of material are placed on the holding plate when it is at its smallest radius so that the speeds match. The plate then extends radially enough during the rotation to match the speed of the plate to the speed of the carrier web. At this point the discrete parts are transferred to the carrier web. This method has two main drawbacks. First, the plate is designed to match the curvature of one radius, not both. This means that either the pick-up of the discrete part or the transfer of the discrete part, or both, will occur across a gap for some part of the transfer. This can lead to a loss of control of the discrete part, which impacts handling of parts under tension, such as leg elastics. Second, to achieve the desired change in speed, the mechanical elements typically used, such as cams or linkages, become fairly large to stay within acceptable design limits for accelerations and rise angles. This size leads to increased cost and reduced flexibility, as the unit must be redesigned for each application.

Another method has utilized noncircular gears to change the speed of a transferring device. The means rotates at a constant radius, but the rotational velocity is varied between a minimum and a maximum to pick up the discrete part at its speed and place the part on the carrier web at its speed. This eliminates the size issues and speed or gap mismatch issues, but relies on mechanical means to achieve the change in rotational velocity. The drawback of this is that new transmission parts (gears or other means) are required each time a change in product design occurs that changes placement pitch length, discrete part length, or other key factors. This can be expensive and time-consuming to change. An example of this method is described in U.S. Pat. No. 6.022,443 issued to Rajala and Makovec. US-5,766,406, by Bahn et al., describes an appparatus for working layer material.

## SUMMARY OF THE INVENTION

In response to the discussed difficulties and problems encountered in the prior art, a new method and apparatus for receiving discrete parts traveling at a speed, changing the speed of the parts to match the speed of a carrier web or body, and applying the parts to the carrier has been discovered.

In one aspect, the present invention concerns an apparatus for receiving discrete parts traveling at a first speed and applying the parts to a carrier traveling at a second speed. The apparatus comprises at least two (rotatable) transferring devices and one driving mechanism for each transferring device. Each rotatable transferring device comprises at least one shell segment configured to move along an orbital path through a receiving zone where the parts are received and an application zone where the parts are applied to the carrier. The carrier might comprise a continuous moving substrate web, or might be another apparatus such as a drum. The driving mechanism utilizes a programmable motor such as a servo motor to transmit rotational energy to the rotatable transferring device. The driving mechanism may transmit rotational energy to the rotatable transferring device through a direct connection or a transmission interposed therebetween. The transmission may include gear to gear contact or gearboxes.

In another aspect, the present invention concerns an apparatus for receiving discrete parts of an elastic material traveling at a first speed and applying the parts to a carrier traveling at a second speed. The parts of the elastic material might already be at the desired tension and elongation length when the apparatus receives them. On the other hand, the parts of the elastic material might be at a lower tension and elongation length than desired when the leading edge is transferred to the apparatus, but might be stretched during the transfer to the apparatus if the apparatus is traveling at a faster speed than the discrete parts. Alternately, the parts might be at a higher tension and elongation than desired when the leading edge is transferred to the apparatus, but can relax to the desired state if the apparatus is traveling at a slower speed than the discrete parts. The change in tension and elongation can be performed uniformly along the entire length of he discrete material part, or can be intentionally varied during the initial transfer by changing the speed of the rotatable transferring means.

In another aspect, the material fed into the transferring device may comprise a continuous web which might be cut into discrete parts while on the transferring device by a knife, hot wire, laser beam, or other method commonly known. Alternatively, the material fed into the system can be broken into discrete parts by acceleration of the rotatable transferring device. This breaking process can be aided by perforating the material at the desired break point prior to arriving at the receiving zone.

In another aspect, the discrete parts can have adhesive or other bonding material applied to them while traveling on the rotatable transferring device. The rotation speed of the transferring device can be controlled in register with a bonding material applicator such that the bonding material can be applied either intermittently or continuously as the rotatable transferring device rotates.

In another aspect, the discrete parts of material can be modified prior to placement on the carrier to aid in a final bonding process. This modification can come through heat addition, moisture addition, or other known method for altering material properties to aid in the bonding process.

In still another aspect, the programmable motors of the apparatus of the present invention can be arranged in a series wherein each of the motors and the transferring devices(s) actuated by each motor is aligned in relation to a common axis of rotation of all motors and transferring devices.

As compared to conventional methods, such as the cut & slip method described above, for changing the speed of a discrete part so that it can be applied to a carrier, utilizing a programmable motor provides the ability to obtain greater changes in speed, to maintain constant speeds for a fixed duration, and to simplify the set-up process when changing from one product to another. Thus, the use of programmable motors can provide a more precise control of the length and placement of the part onto the carrier while offering great flexibility in the type of parts that are to be made.

## BRIEF DESCRIPTION OF THE DRAWINGS

The present invention will be more fully understood and further advantages will become apparent when reference is made to the following detailed description of the invention and the accompanying figures. The figures are merely representative and are not intended to limit the scope of the appended claims.

Figure 1 representatively shows an isometric view of an apparatus outside the scope of the present invention.
Figure 2 representatively shows a schematic side view of the apparatus in Figure. 1.
Figure 3 representatively shows a schematic side view of an apparatus arranged in series.

Figure 4 representatively shows a perspective view of an apparatus according to the present invention comprising two transferring devices.

Figure 5 representatively shows a perspective view of the apparatus shown in Figure 4 including a cutting device.

Figure 6 representatively shows a speed profile for a typical motor output.

Figure 7 representatively shows an alternate speed profile for a motor output wherein one of the fixed speed regions has been changed to variable speed.

Figure 8A representatively shows an alternate speed profile for a motor output wherein the rise time β has been decreased to allow for a non-optimal radius without changing the maximum or minimum rotational velocity in the system.

Figure 8B representatively shows an alternate speed profile for a motor output wherein the maximum rotational velocity during the period of transition from receiving to application zone exceeds the average rotational velocity in the application zone to allow for a non-optimal radius.

Figure 8C representatively shows an alternate speed profile for a motor output wherein the minimum rotational velocity during the period of transition from receiving to application zone is less than the average rotational velocity in the receiving zone to allow for a non-optimal radius.

Figure 9a representatively shows a perspective view of an apparatus according to the present invention including an applicator for accomplishing a secondary process on the parts, and two transferring devices comprising multiple shell segments.

Figure 9b is a side view of the apparatus shown in Figure 9a.

Figure 10 representatively shows an isometric view of adjacent shell segments according to the present invention having grooved leading and trailing edges that mesh.

Figure 11 representatively shows an isometric view of another embodiment of the present invention including a series of motors aligned with a common central axis and wherein each of the motors has a hollow rotatable shaft.

Figure 12 representatively shows a plan view of the embodiment shown in Figure 11.

Figure 13 representatively shows an isometric view of the embodiment shown in Figures 11-12 in combination with a cutting device as viewed from upstream.

Figure 14 representatively shows an isometric view of the embodiment shown in Figures 11-12 in combination with a cutting device as viewed from downstream.

Figure 15 representatively shows a side view of the embodiment shown in Figures 13 and 14.

Figure 16 representatively shows an isometric view of another embodiment of the present invention including a series of motors aligned with a common central axis and wherein each of the motors has at least one stationary track rail and a movable rider.

Figure 17 representatively shows a plan view of the embodiment shown in Figure 11.

Figure 18 representatively shows a side view of the embodiment shown in Figures 16 and 17.

Figure 19 representatively shows an isometric view of another embodiment of the present invention including a series of motors aligned with a common central axis and wherein each of the motors has a stationary inner stator and a rotatable outer rotor.

Figure 20 representatively shows an isometric view of another embodiment of the present invention including a series of motors aligned with a common central axis and wherein each of the transferring devices follows an orbital path.

Figure 21 representatively shows a plan view of the embodiment shown in Figure 20.

Figure 22 representatively shows a side view of the embodiment shown in Figures 20 and 21.

Figure 23 representatively shows an isometric view of the embodiment shown in Figures 20-22 including a cutting device as viewed from downstream.

Figure 24 representatively shows an isometric view of the embodiment shown in Figures 20-22 including a cutting device as viewed from upstream.

Figure 25 representatively shows a side view of the embodiment shown in Figures 23-24.

Figure 26 representatively shows an isometric view of another embodiment of the present invention including a series of motors aligned with a common central axis and wherein each of the motors has at least one stationary planetary track rail and a movable rider.

Figure 27 representatively shows an isometric view of another embodiment of the present invention including a stretching device.

Figure 28 representatively shows an isometric view of another embodiment of the present invention including a rotating device.

DETAILED DESCRIPTION OF THE INVENTION

The present invention provides a method and apparatus for receiving discrete parts traveling at a first speed and applying the parts to a carrier traveling at a second speed. The apparatus and method are particularly useful for applying any part to a carrier useful in the making of disposable absorbent articles or for placing labels onto articles. It is readily apparent, however, that the method and apparatus would be suitable for applying any part to a substrate web or carrier of parts.

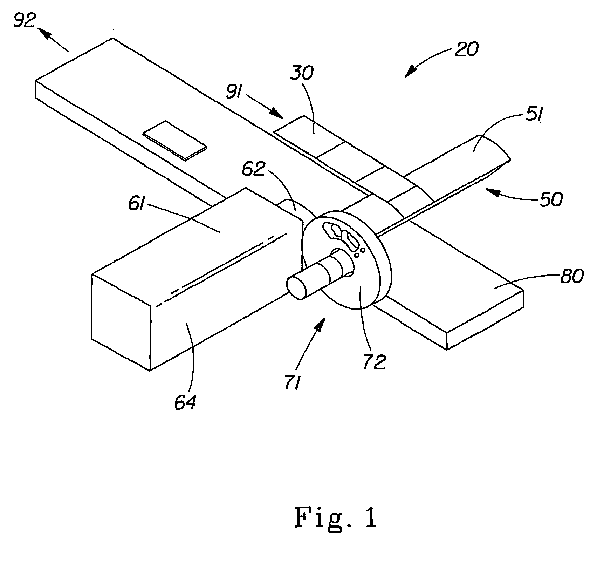

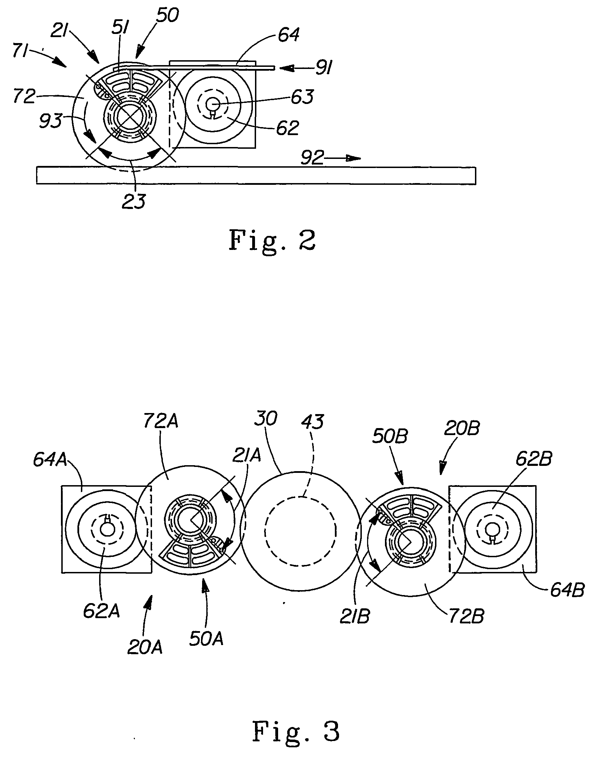

Referring now to Figures 1 and 2, there is representatively shown an aspect outside the scope of the invention wherein an apparatus generally indicated at 20 receives discrete parts 30 traveling at a first speed in the direction indicated by the arrow 91 associated therewith and applies the parts 30 to a carrier 80 traveling at a second speed in the direction indicated by the arrow 92 associated therewith. The illustrated example of the apparatus 20, as representatively shown in Figures 1 and 2, further comprises a driving mechanism 61 for transmitting rotational energy to a driven mechanism 71. The driving mechanism 61 includes a connection to the driven mechanism using any technique known to those skilled in the art such as, for example, gear to gear connection, transmission belting and pulleys, gearboxes, direct couplings, and the like or any combinations thereof. For example, in Figure 1 the driving mechanism is connected to a driving gear 62 which transmits rotational energy to a driven gear 72 connected to the driven mechanism 71. In use, the driving gear 62 engages and rotates the driven gear 72 which, in turn, rotates the transferring device 50.

The illustrated example of the transferring device 50 comprises at least one shell segment 51 connected to the driven mechanism 71. The shell segment 51 of the transferring device 50 can be connected to the driven mechanism 71 by any technique known to those skilled in the art such as, for example, bolts, screws, pins, keys and matching key ways, connector parts such as shafting or brackets, welding and the like or combinations thereof. For instance, the shell segment 51 shown in Figure 1 is connected directly to the driven gear 72 by fitting the end of the shell segment 51 into a mating hole in the driven gear 72 and locking it into position with a pin. Similarly, other components of the apparatus 20 can be connected together employing the above described assembly techniques.

The dimensions of the shell segment 51 may vary depending upon the desired output of the apparatus 20 and the size and shape of the discrete articles 30 being transferred. The shell segment 51 may comprise a crescent-shaped member having an outer, peripheral arc length spanning from about 5 degrees to about 340 degrees, an outer radius ranging from about 25 mm to about 500 mm, and a width ranging from about 50 mm to about 750 mm. As the driven mechanism 71 rotates, the transferring device 50 travels in the direction indicated by the arrow 93 as shown in Figure 2. The circumferential, outer peripheral surface of the shell segment 51 defined by an outer radius , travels along and defines an orbital path that passes through a receiving zone 21 and an application zone 23. The receiving zone 21 and the application zone 23 are defined by the respective regions of the orbital path traveled by the shell segment 51.

The illustrated example of the driving mechanism 61 includes a rotatable circular driving gear 62 connected to an input shaft 63. In this example, the input shaft 63 is the output shaft of the motor 64. The driven mechanism 71 is placed parallel to the driving mechanism 61 such that the driving gear 62 meshes with the driven gear 72 using gear set-ups known to those skilled in the art. In use, the motor 64 rotates the input shaft 63 which rotates the driving gear 62 which, in turn, rotates the driven gear 72 and transferring device 50.

Alternatively, the driven mechanism 71 may include any mechanism known to those skilled in the art by which rotational energy can be conducted from one shaft to another such as, for example, v-belts, timing belts, continuous chains and the like or combinations thereof. Further, the driven mechanism 71 may include any mechanism known to those skilled in the art by which input velocity can be variably modified to an output source such as, for example, cams, linkages, and the like or combinations thereof as long as the changes in rotational speed are substantially created by the motor 64.

It will be further appreciated that the method and apparatus 20 of the invention can utilize two, three or more combinations of transferring devices 50, driven mechanism 71, driving mechanism 61 and motor 64 in series to achieve the desired application of the discrete parts to the carrier. The different combinations may allow the use of a continuously moving web to supply the discrete parts. In addition, greater speed ratio differentials may be achieved by using combinations of transferring devices, driven mechanisms, driving mechanisms and motors in series.

It will be further appreciated that the method and apparatus 20 of the invention, when used in series, do not need to operate at the same receiving zone 21 and application zone 23.

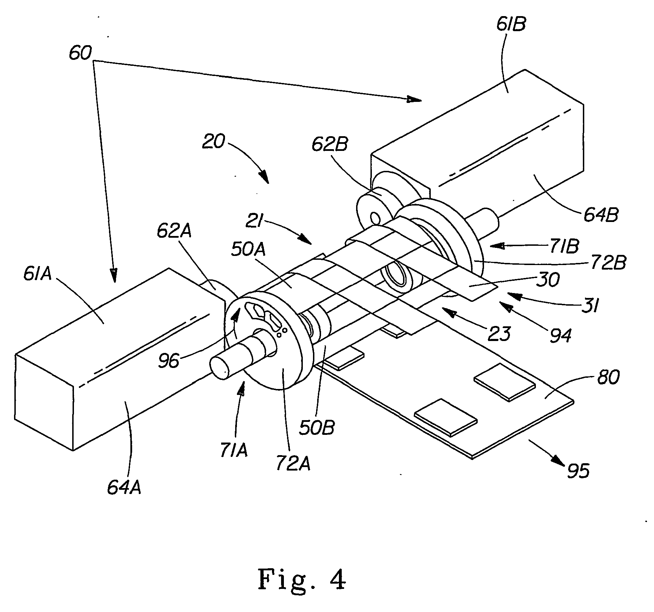

Another aspect of the invention shown in Figure 4 comprises an apparatus 20 receiving discrete parts 30 of a web of an material 31 traveling at a first speed in the direction indicated by the arrow 94 associated therewith and applies the parts 30 to a carrier 80 traveling at a second speed in the direction indicated by the arrow 95 associated therewith. The illustrated example of the apparatus 20 comprises two rotatable transferring devices, represented by 50A and SOB, for receiving and applying the parts 30. The apparatus 20 further comprises a driving system 60 having two driving mechanisms 61A and 61B, each of which includes a motor 64A, 64B and a driving gear 62A, 62B for transmitting rotational energy to the driven mechanism 71A, 71B represented by the driven gear 72A, 72B.

As illustrated in Figure 4, each transferring device 50A and SOB comprises a shell segment 51A, 51B connected to a driven gear 72A, 72B. As each gear rotates, the transferring devices 50A, 50B travel in the direction indicated by the arrow 96 associated therewith. In use, the circumferential, outer peripheral surface of the shell segments 51A, 51B travels along and defines an orbital path that passes through a receiving zone 21 and an application zone 23 defined by the respective regions of the orbital path traveled by the the shell segments 51A, 51B of transferring devices 50A and 50B.

The size and shape of the shell segments 51A and 51B may vary as the number of shell segments per transferring device changes. For example, if the apparatus includes two transferring devices as representatively illustrated in Figure 4, each shell segment 51A and 51B may have an outer peripheral arc length which spans from about 5 to about 175 degrees of the orbital path of the transferring devices 50A and 50B.

Each driven mechanism 71A, 71B may include any mechanism known to those skilled in the art by which rotational energy can be conducted from one shaft to another such as, for example, v-belts, timing belts, continuous chains and the like or combinations thereof. Further, the driven mechanisms 71A, 71B may include any mechanism known to those skilled in the art by which input velocity can be variably modified to an output source such as, for example, cams, linkages, and the like or combinations thereof as long as the changes in rotational speed are substantially created by the motor 64. Alternatively, a first driven mechanism may connect to a first transferring device using a first shaft from a driven gear, and a second driven mechanism may be connected to a second transferring device using concentric shafting around the first shaft.

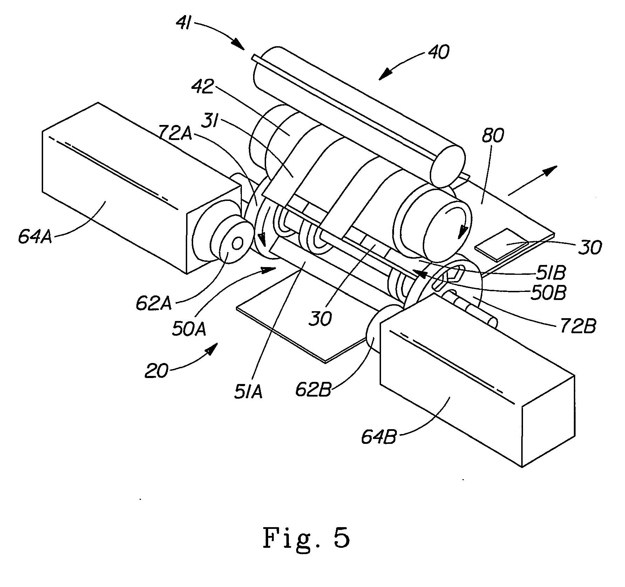

The apparatus 20, as representatively illustrated in Figure 5, may further comprise a cutting device 40 comprising a pinch knife cutter 41 and a knife anvil 42 to sever the continuously moving web of material 31 into discrete parts 30 prior to or concurrent with the transfer of the discrete parts to the shell segments 51A, 51B of the transferring devices 50A, 50B. The pinch knife cutter 41 may comprise a rotary cutter as shown or any other mechanism known to those skilled in the art capable of severing a web of material into discrete parts. In certain aspects of the invention, the knife anvil 42 may be omitted and the pinch knife cutter 41 can be made to sever the web as it is held on the shell segment of the transferring device. Alternately, the continuously moving web of material may be placed directly on the shell segments of multiple transferring devices so that the web lies on multiple segments at once allowing for the planned acceleration of one device to generate a force necessary to sever a single part from the web. Such severing may be facilitated by perforating the web upstream of the receiving zone so that the parts break at a desired perforation during acceleration.

For receiving the parts in the receiving zone, the transferring device, as representatively illustrated in the various configurations of the invention, may further include a gripping mechanism so that the outer concave surface of the shell segment can capture a part in the receiving zone and transport the part to the application zone. For this embodiment, the gripping mechanism may include a vacuum that can be selectively imposed through ports in the shell segment leading to the outer concave surface. For instance, the vacuum may be activated in the receiving zone to seize the parts and deactivated in the application zone to release the parts to the carrier. In this manner, positive control is maintained over the parts at all times during the transfer process. Alternatively, the gripping mechanism may include any technique known to those skilled in the art for gripping and releasing parts such as, mechanical clamps, electrical clamps, magnetic clamps and the like or combinations thereof.

For transferring the parts to the carrier in the application zone, the apparatus may comprise any of a variety of options known to those skilled in the art such as, adhesive applied on the part, adhesive applied on the carrier, electrostatic charge between the part and carrier, vacuum on the carrier and the like or combinations thereof. Alternately, the transfer can include the generation of a weld between the part and the carrier by any of a variety of means known to those skilled in the art such as, pressure generation at a nip formed between the shell segment and the carrier at transfer, interaction between a pattern on the shell segment and an ultrasonic horn behind the carrier at transfer, and the like, or combinations thereof. In addition, in order to aid the welding process, the part may be modified on the shell segment by energy addition using any mechanism known to those skilled in the art such as, for example, hot air currents, ultraviolet lighting, laser bombardment and the like or combinations thereof.

The use of a programmable motor in the apparatus, as representatively illustrated in the various aspects of the invention described above, provides an inexpensive and adaptable method for receiving parts 30 traveling at a speed and applying the parts to a carrier 80 traveling at a different speed. The variable angular velocity is produced by varying the current supplied to the motor. Since the driven mechanism is coupled to the output of the motor, changes in the angular velocity and position of the motor directly correlate to changes in the angular velocity and position of the transferring device. The current supplied to the motor can be controlled using any of a variety of a methods for programming motors known to those skilled in the art such as, standard cam curve functions, a reference data table containing reference points, desired motor encoder points, and the like or combinations thereof.

The means of supplying the rotational movement required can be achieved in a plurality of methods to those skilled in the art. The programmable electric motors can be driven from any known power source that is capable of delivering a modulated signal such that the motor torque can be varied proportionally. The number of motors included per a transferring device can be any suitable number. Each motor attached to a single transferring device can be supplied by one or more power sources capable of delivering a modulated torque signal. The torque signal is typically an electrical current which may be fed to the individual motors by separate power supplies or by a single power supply and controlled by a plurality of methods to those skilled in the art.

The actual position of the transferring device can be controlled in a plurality of methods to those skilled in the art. The control system demonstrated utilizes a programmable system that incorporates a position feedback from the transferring device and motor. The position feedback device on the transferring device, or motor, may or may not be required if the position of the transferring device can be inferred by other means known to those skilled in the art. The typical position transducer is an encoder, or resolver based system, but any system that can be constructed to provide the actual position, or the inferred position of the transferring device can be used.

The demonstrated control system is one of many kinds, models, and technologies that can be used to supply the proportional signal to the motor power supplies that will generate the modulated torque signal.

The control system may or may not be integrated into the motor power supply. The control system, along with the motor power supply, may or may not be integrated into the motor itself. The control system may or may not be digitally controlled, and may be constructed in various methods, and configurations known to those skilled in the art. The control system, power supplies, feedback devices, and motor devices, and any other components required for the purpose of providing rotational movement are hereafter referred to as the "drive system" for the transferring device.

The drive system will be capable of continuously controlling the position of the transferring device, and allowing the transferring device to stay in phase to a given position on the recipient product, web, or host machine. The drive system will be capable of following speed transitions or position variations on the recipient product or web, by phasing itself, when necessary, to the recipient product, web, or host machine, with or without operator intervention. The drive system will allow for the registration of the patch on the transferring device in relation to the recipient product or web, either upstream or downstream of the transferring device.

The drive system may be capable of providing for a plurality of control methods and algorithms known to those skilled in the art for the purpose of providing motion and position control that will allow the transfer of a patch to a recipient product or web. The drive system may be capable of changing the patch length with or without operator intervention, for the purpose of varying product sizes or continuous patch length, or position variation control. The position reference for the drive system may be a pre-calculated cam profile, continuously calculated profile, or any positional trajectory generation algorithm known to those skilled in the art, and may be either digital or analog based. The motion trajectory for the transferring device may be based on a pre-calculated profile or a profile that is modified by the speed of the recipient product or web.

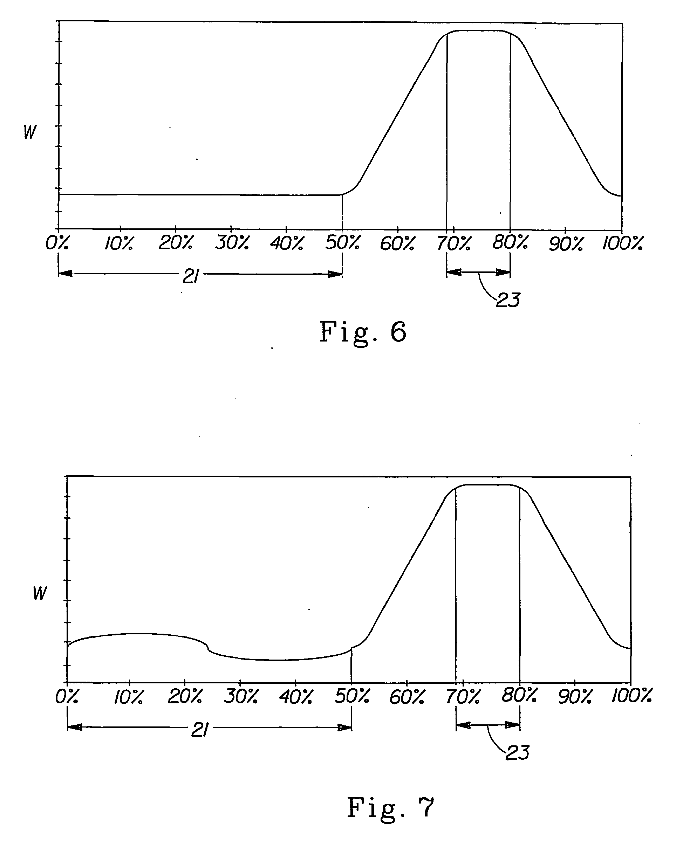

The speed profile of a typical motor setting is representatively illustrated in Figure 6. As shown, the programmable motor 64 used to drive the transferring device 50 of the present invention can provide variable angular velocities including periods where the velocity remains constant for a fixed duration. These constant velocity dwell times can be advantageous in the receiving zone 21 and the application zone 23 particularly when the pick up and transfer occurs over substantial arc lengths of contact. Alternatively, one or more of the constant speed regions can be changed to a controlled variable speed region as representatively illustrated in Figure 7. This would enable the part 30 to be picked up in the receiving zone 21 at a variable speed, which, when the part 30 is elastic, would allow tensions to be varied incrementally therein which may be desirous in certain product features. In another example, the constant speed of the motor 64 in the application zone 23 can be such that the corresponding speed of the transferring device is different from speed of the carrier at transfer. Such speed variations generate tension in the part 30 by incrementally transferring the part 30 in a controlled manner from one means traveling at one surface speed to a second means traveling at a second surface speed.

It will be further appreciated that the velocity of the transferring device 50 outside of the application zone or the receiving zone can be tailored to aid the performance of secondary processes including adhesive application, printing of identification or registration marks, application of bonding aids, moisture addition and the like and combinations thereof. Such changes in velocity may be beneficial by presenting specific velocity profiles or even additional periods of constant velocity, which would allow for more precise interaction with the secondary processes being performed.

Programmable motors, such as those used in the present invention, can be purchased from any number of suppliers of programmable motors such as Rockwell Automation, located in Milwaukee, Wisconsin. Further, the program inputs to the motors can be generated by one of ordinary skill in the art if provided with the analytical representation of the desired output function as representatively illustrated in Figure 6. For instance, the creation of the electronic cam profile for the motor can be developed by first determining the key input variables. The key input variables are based on desired product features, the base design of the apparatus 20 and the desired cycle speed of the apparatus 20. Secondly, the radius of the outer surface of the transferring device 50 is determined. Once the radius is determined, the required cam inputs of rotational velocities, distances traveled and time available for acceleration can be calculated, which serve as the input to the cam profile generator. For example, in a system with the following inputs:

$N$ = the number of transferring devices 71 used in the apparatus 20

$H$ = the number of shell segments 51 per transferring device 71

$L_{s,part}$ = distance from lead edge of first part 30 received in a given transfer to transferring device 50 to lead edge of part 30 received in the next cycle of transfer to a transferring device 50 in the apparatus 20

$L_{s,product}$ = distance from lead edge of first product zone on carrier 80 to which parts 30 are applied in a given transfer from transferring device 50 to lead edge of product zone on carrier 80 to which parts 30 are applied in the next cycle of transfer from a transferring device 50 in the apparatus 20

$V_{min}$ = average surface speed of the shell 51 on the transferring device 50 in receiving zone 21

$V_{max}$ = average surface speed of the shell 51 on the transferring device 50 in application zone 23

$\tau$ = cycle time of a given lane of product making

$\tau_R$ = time in receiving zone 21, typically of value $\tau$ whey $V_{min}$ = incoming speed of parts 30

$\tau_A$ = time in application zone 23, typically of value Ratio *$\tau$ when $V_{max}$ = speed of carrier 80

The following dependent variables can be computed:

$$Ratio = L_{s,part} / L_{s,product}$$

Radius = distance from effective center of rotation of transferring device 50 to outer surface of shell 51 on transferring device 50

$$\tau_{TRANS} = \text{time in transition from } V_{min} \text{ to } V_{max} = N * \tau - \tau_R - \tau_A = ( N - 1 - Ratio) * \tau$$

$$\omega_{min} = \text{average angular velocity of transferring device 50 in receiving zone 21} = V_{min} / Radius$$

$$\omega_{max} = \text{average angular velocity of transferring device 50 in application zone 23} = V_{max} / Radius$$

$$\theta_{min} = \omega_{min} * \tau_R = \omega_{min} * \tau$$

$$\theta_{max} = \omega_{max} * \tau_A = \omega_{max} * Ratio * \tau$$

$$\theta_{transition} = 2 * \pi / H - \theta_{min} - \theta_{max} = 2 * \pi / H - \omega_{min} * \tau - \omega_{max} * Ratio * \tau$$

The Radius may be determined by assuming that the average angular velocity, $\omega_{ave}$, of the transferring device 50 during the transition from the receiving zone 21 to the application zone 23 is equal to $(\omega_{min} + \omega_{max}) / 2$. This means that the distance traveled during the transition $\theta_{transition} = \omega_{ave} * \tau_{Trans}$. However, $\theta_{transition}$ must also be equal to $2 * \pi / H - \theta_{min} - \theta_{max}$. Consequently, by setting the two equations for $\theta_{transition}$ equal to one another the following expression for Radius is defined.

$$Radius = (L_{s,part} * ( N + 1 - Ratio ) + L_{s,product} * ( N - 1 + Ratio ) ) * H / (4 * \pi)$$

It is important to note that $L_{s,part}$ as defined can be different from the overall length of the part, for example, when the edges of the part are cut at some angle which is not perpendicular to the direction of flow of the part. The above equations and parameters must be solved slightly differently to account for this.

Now, given the inputs, one of ordinary skill can determine $\tau_{TRANS}$, $\omega_{min}$, $\omega_{max}$, $\theta_{min}$, $\theta_{max}$, and $\theta_{transition}$ which are typical inputs needed for electric cam software programs. The generic cam programs would then create the input table for the motor 64. Note that the Radius is an optimal radius, and not the only possible radius for the set of inputs. The Radius is optimal because it uses the entire transition time for changing the angular velocity of the transferring device 50. By changing the Radius, the actual amount of time required to change speed must change or else the combined conditions of change in angular velocity and change in angular acceleration will not be met. The amount by which the Radius can deviate from the optimum can be changed from optimal depends upon the torque requirements of the system under the new accelerations at the given speed and the capability of the selected motor 64.

Alternately, one of ordinary skill could generate the input table for the motor without the aid of software programs. For example, the cam profile for cycloidal motion having dwells of constant velocity comprises a minimum velocity equal to $\omega_{min}$, a change in velocity, $\Delta\omega$, equal to $\omega_{max} - \omega_{min}$, and a rise time $\beta$ of the motion equal to $\tau_{TRANS}/2$. The resulting function of angular position is as follows:

$$\theta_{act} = \omega_{min} * T \text{ when } 0 \leq \text{time in cycle, } T \leq \tau_R$$

$$\theta_{act} = \omega_{min} * T + \tfrac{1}{2} * \Delta\omega * \beta * (\,(T - \tau_R)/\beta - \sin(\,(T - \tau_R)*\pi/\beta\,)/\pi\,) \text{ when } \tau_R < T < \tau_R + \beta$$

$$\theta_{act} = \omega_{min} * T + \tfrac{1}{2} * \Delta\omega * \beta + \omega_{max} * (T - \tau_R - \beta) \text{ when } \tau_R + \beta \leq T \leq \tau_R + \beta + \tau_A$$

$$\theta_{act} = \omega_{min} * T + \tfrac{1}{2} * \Delta\omega * \beta + \omega_{max} * \tau_A + \tfrac{1}{2} * \Delta\omega * \beta * (\,(T - \tau_R - \beta - \tau_A)/\beta - \sin(\,(T - \tau_R - \beta - \tau_A)*\pi/\beta\,)/\pi\,) \text{ when } \tau_R + \beta + \tau_A < T \leq \tau$$

Profiles other than the cycloidal profile can be found in Machinery's Handbook, the 25th Edition.

If a radius is chosen for the transferring device that is not optimum, the transferring device will accomplish the desired changes in velocities, however, the timing for such changes will not correspond to that which is desired. For instance, if the Radius is slightly greater than optimal, using the equations above, the actual distance traveled during the transition is less than needed to position the transferring device 50 at the start of the application zone 23 even though the transferring device 50 achieves the desired angular velocity.

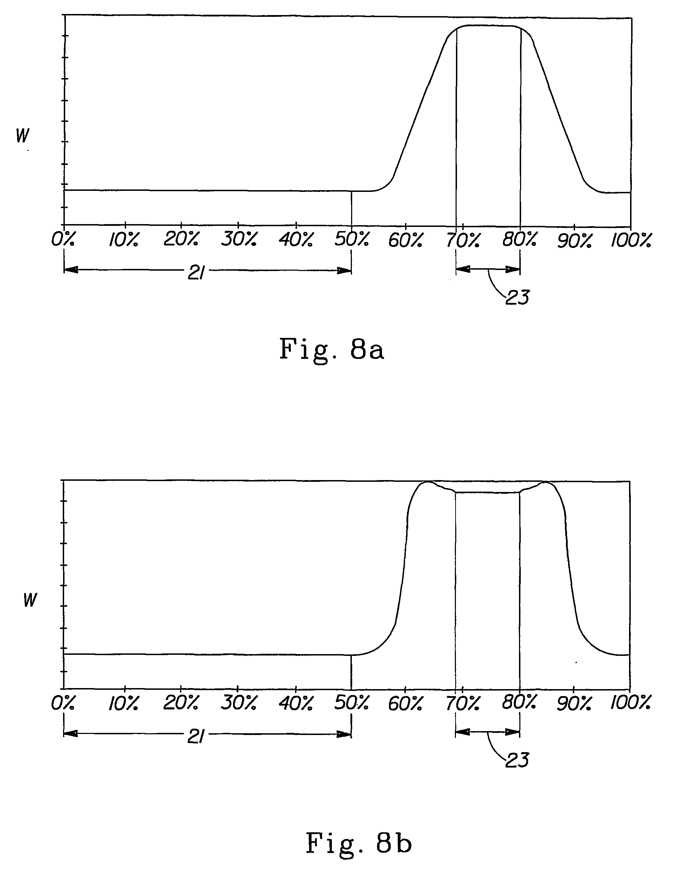

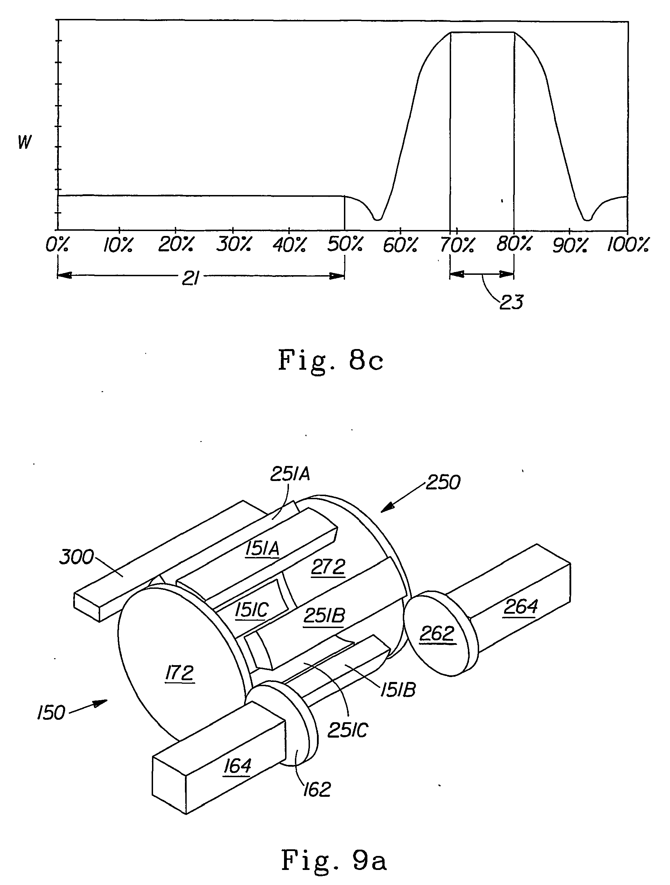

There are at least three possible ways to achieve speed profiles accommodating pick up and transfer using a non-optimal Radius. First, the rise time $\beta$ can be decreased by spending more time at $\omega_{min}$ as shown in Figure 8A. Secondly, as shown in Figure 8B, the maximum angular velocity in the transition zone can be greater than the $\omega_{max}$ in the application zone 23. Thirdly, the minimum angular velocity in the transition zone can be less than the $\omega_{min}$ in the receiving zone 21 as shown in Fig. 8C.

Using the same cam formulas, one can determine the maximum accelerations generated during the motion using the same family of cam profiles. For example, for the cycloidal profile used above, the peak acceleration is $(\Delta\omega) * \pi)/(2 * \beta)$. This is important because, for high speed applications, the limiting design factor in the apparatus 20 is motor 64 torque capability at the desired angular velocities. One of ordinary skill in the art can determine total torque requirements for the apparatus 20 for a given application based on the masses and radii of gyration for the different components of the apparatus 20 and the expected accelerations.

As compared to conventional methods for changing the speed of a discrete part so that it can be applied to a continuously moving carrier (such as the slip cut method described above), the use of programmable motors provides the ability to obtain greater changes in speed and to maintain constant speeds for a fixed duration. The fixed speed dwell achieved by programmable motors can be accurately and quickly generated to control the length and placement of the parts. In comparison to the noncircular gear method described above, the use of programmable motors provides the ability to change the profile at will without requiring the fabrication of new parts.

For example, in the various aspects of the invention, the profile generated by the programmable motor 64 is analytically designed such that the rotatable transferring device 50 receives the parts 30 in the receiving zone 21 while maintaining a constant surface speed substantially equal to the speed of the parts 30. Moreover, the output profile of the motor 64 is designed such that the surface speed of the rotatable transferring device 50 changes to a second constant surface speed as the rotatable transferring device 50 moves from the receiving zone 21 to the application zone 23. The term "surface speed," as used herein, refers to the speed of the circumferential, outer peripheral surface of the shell segment 51. The output profile of the motor 64 can be designed such that the speed of the parts 30 being transferred is substantially equal to the speed of the carrier 80 as the parts are applied to the carrier in the application zone 23. The surface speed of the shell segment 50 is maintained substantially constant in the receiving zone 21 and in the application zone 23 from at least about 0 to about 300 degrees of rotation, desirably from about 5 to about 300 degrees of rotation, and more desirably from about 5 to about 240 degrees of rotation of the transferring device 50. In addition, the surface speed increase or decrease of the shell segment 51 as it moves from the receiving zone 21 to the application zone 23 defines a speed ratio of from at least about 100:99 to about 50:1, desirably from about 20:19 to about 25:1, and more desirably from about 10:9 to about 20:1. The term "speed ratio", as used herein, defines the ratio of the surface speed of the shell segment 51 as the parts 30 are applied to the carrier 80 in the application zone 23 to the surface speed of the shell segment 51 as the parts 30 are received in the receiving zone 21.

It has been described above how the required torque and angular speed of the apparatus determine the needed motor capability. For high speed applications common in the manufacture of articles such as diapers, training pants, among other uses, the peak torque requirements of the apparatus 20 combined with the resulting acceleration at the motor 64 will require very high torque to inertia properties in the motor 64. Motors capable of such flux densities are typically of rare earth permanent magnet design or more powerful, and can be purchased from manufacturers of motors such as Rockwell Automation located in Milwaukee, Wisconsin.

In some embodiments it may be necessary to have more than one shell segment per transferring device driven by a single motor, particularly where the process includes secondary operations that are preferably performed at constant speed (see below). It may also be necessary to have multiple shell segments per transferring device in order to increase the radius from the center of rotation to the arcuate outer surface of the shell segments. The radius of a transferring device having a single shell segment may be so small that secondary parts of the design (such as porting airflow for vacuum across the shell segment) may be impractical. Based on the equation above for determining Radius, adding shell segments per transferring device results in an increase in Radius. For example, increasing the number of shell segments per transferring device from one to three triples the Radius.

Additional shell segments also result in an increase in motor torque which is determined from the following expression.

$$T = I_{motor} * \alpha_{motor} + I_{load} * \alpha_{motor} * (N_{driving}/N_{driven})^2$$

where

$I_{motor}$ - is the moment of inertia of the motor including anything connected directly to the motor shaft.

$\alpha_{motor}$ - is the angular acceleration of the motor

$N_{driving}/N_{driven}$ - the ratio of the number of teeth on the driving gear to the number of teeth on the driven gear.

$I_{load}$ - is the moment of inertia of the load (including the transferring device).

The additional shell segments result in an increase in mass moment of inertia of the transferring device, $I_{load}$. Inertia is a direct function of mass and radius of gyration squared. For a transferring device having three shell segments, the mass can be expected to approximately triple and the radius of gyration can be expected to nearly double. Consequently, the moment of inertia of the transferring device can be expected to be at least ten to twelve times the inertia of an equivalent system having a single shell segment. The increase in inertia results in a decrease in the rotational velocity by a factor of three in a system having three shell segments per transferring device, consequently, the gear ratio, $N_{driving}/N_{driven}$ is increased by a factor of three in order to maintain the required motor acceleration. Overall; the resultant torque requirement for the system having three shell segments per transferring device is 11% greater relative to a transferring device having one shell segment per transferring device. Given that motor capability is the key limiting design factor, an increase of 11% in required torque can be significant and can potentially limit the capacity and application of the apparatus.

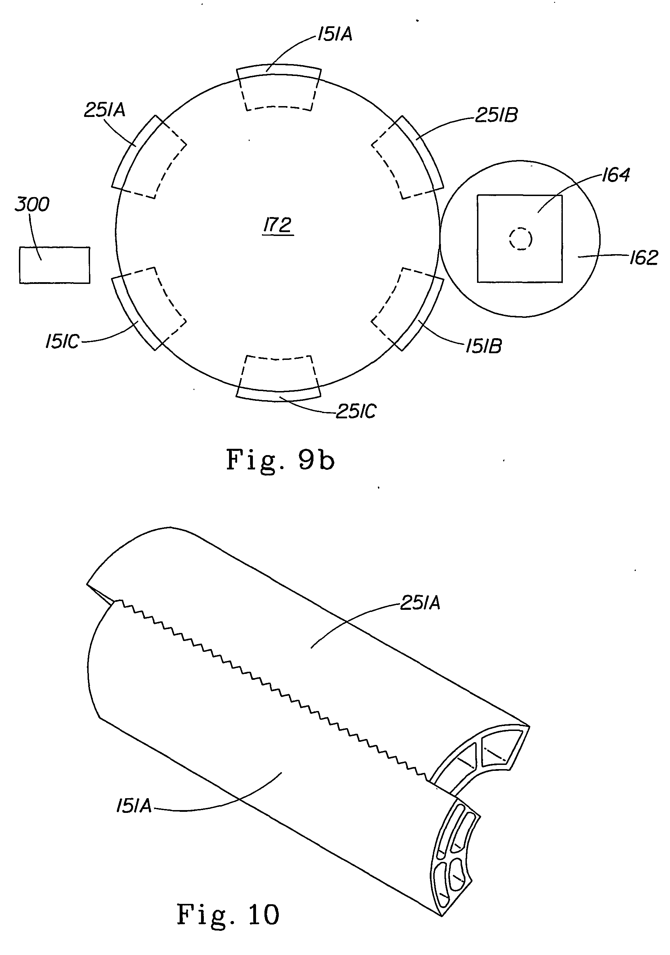

There is no restriction on the number of shell segments per motor besides space and inertial concerns, however, the arrangement pattern of multiple devices is limited. For instance, a transferring device having two shell segments per motor cannot be arranged such that any two shell segments on one transferring device are adjacent to one another in sequence without at least one shell segment from a separate transferring device driven by a separate motor interposed between them. Figures 9a and 9b portray an apparatus according to the present invention including an applicator for performing a secondary process on the parts and two transferring devise150 and 250, each having multiple shell segments. Transferring device 150 comprises three shells 151A, 151B, and 151C and transferring device 250 comprises three shells 251A, 251B, and 251C. Each transferring device is driven by a separate motor 164,264. As shell segment 151A of transferring device 150 collects a part in the receiving zone 21, the surface speed of shell segments 151A, 151B and 151C are each equal to the receiving speed while the surface speeds of shell segments 251A, 251B, and 251C of transferring device 250 are each equal to either the application speed or some other transitional speed.

Transferring devices comprising two or more shell segments are particularly beneficial where the process includes secondary processes where it is necessary for the part to be moving at a constant speed. As previously described, a secondary process step can be performed on the part such as adhesive application, printing, heating, or moisture application at any point between the receiving zone and the application zone. However it is preferred to perform the secondary process while the part is moving at constant speed. In applications where the parts are received as a continuous web with no scrap separating the parts during separation, it is preferred that no gaps occur between adjacent shell segments during the transfer in the receiving zone. For instance, where the secondary process involves the application of an adhesive, it is preferred that the parts move at a constant speed during the application with no gaps in between parts so that the metering rate of the adhesive can be constant and not intermittent.

In order to perform a secondary process on the part at a constant speed with no gaps, the secondary process can be performed on the part before the transferring device starts to accelerate. In other words, the time at the receiving speed is lengthened so that the full arc length of the shell segment of the transferring device (assuming that the entire length of the part is required) passes through the process. For example, if the process involves application of an adhesive, then the entire arc length passes underneath the nozzle of the adhesive applicator. This presents a challenge for a transferring device comprising a single shell segment driven by a single motor since the sum of the receiving time, the application time, and the time for the secondary process might exceed the total cycle time. This problem may be resolved by adding a second transferring device having a single shell segment driven by a separate motor or by using a transferring device having more than one shell segment per transferring device.

In the process, generally, as one shell segment finishes receiving a part, the next shell segment in sequence starts receiving a part. Generally, the parts entering the transferring device are part of a continuous web having no spacing in-between at the receiving zone. Consequently, in order to have a no gap situation, shell segments in the receiving zone should have no gap between them. However, in reality, variability in motor positioning, head dimensions, gear backlash, etc. can cause the position of the transfer device to have some variability in rotational position resulting in gaps or even forcible contact therebetween. The above problem can be resolved by the embodiments of the present invention described hereinbelow.

First, the shell segments can be fabricated to be a little shorter in arc length than the part to be received, and then the devices can be brought close but not contacting each other. This avoids contact, but does not allow for complete control of the lead and trail edges of the part. This may or may not impact product quality, depending on the part properties. Second, the shell segments can have a shock-absorbing structure on the lead and / or trail edges, so that contact does not generate damage. Depending on the desired cycle life of the transfer device, this might be as simple as placing a compressible foam or as complex as a spring-loaded wall. Third, the shell segments can have mating surfaces which allow the devices to coexist radially and tangentially but not axially. As shown in Figure 10, shell segments 151A and 215A can have grooved or otherwise modified leading and trailing edges that mesh with similarly modified edges on adjacent shell segments.

It should be understood by one skilled in the art that the embodiments of the present invention having a driven mechanism placed parallel to a driving mechanism described hereinabove are limited to a number of transferring devices and/or a number of heads per a transferring device and/or a number of independent motors per a transferring apparatus. First, this is due to the increasing mechanical complexity associated with providing sufficient stiffness for the shafts rotating the transferring devices because in order to provide more than two rotating transferring devices sharing the same axis of rotation per an apparatus there will be more than one rotating transferring device per a side of the carrier web and the shafts driving the transferring devices located on the one side of the carrier web will be arranged concentrically to each other and thus, the diameters and/or wall thicknesses of the concentric shafts will diminish as the number of transferring devices on the one side of the carrier web increases. Second, as described therein above, as the number of shell segments per a transferring device increases, the torque requirements for the system increases. Third, the number of independent motors driving a transferring device is limited by the physical space available around the transferring device.

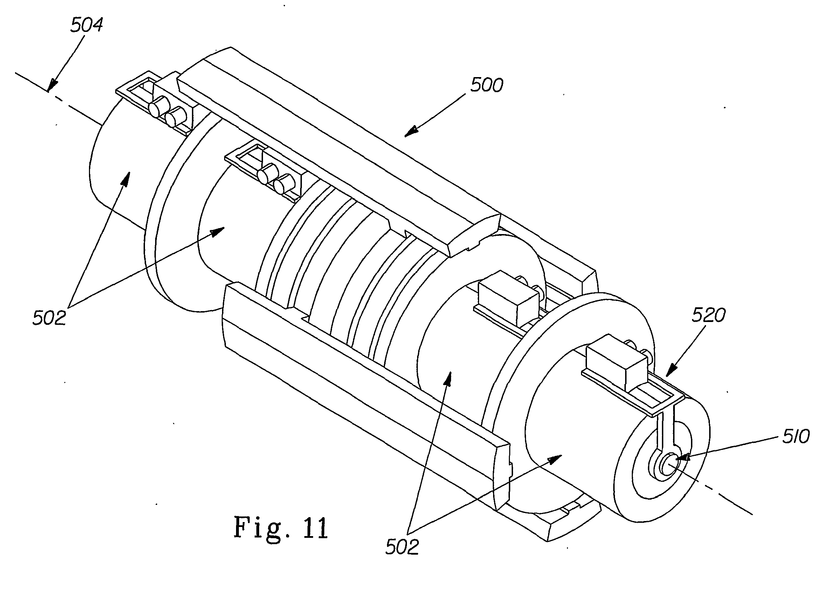

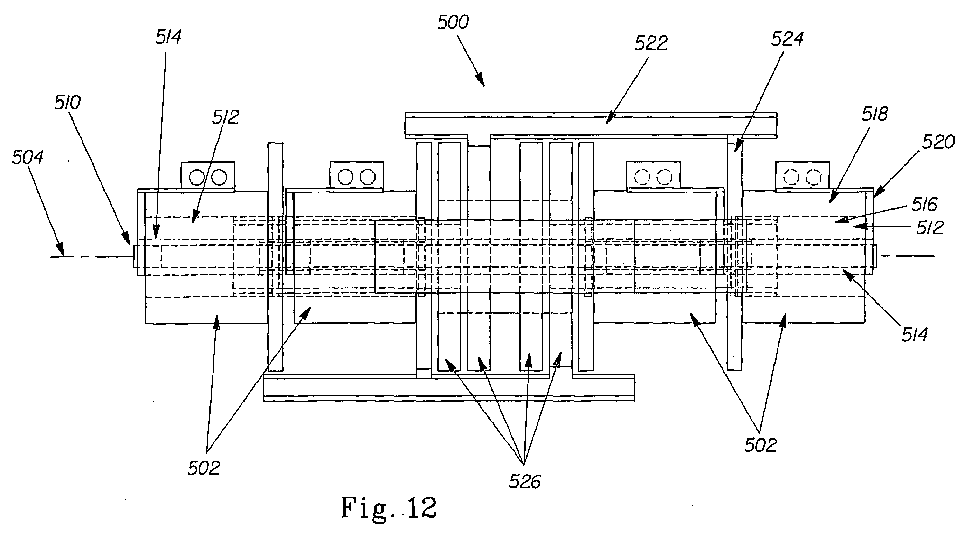

Referring now to Figure 11-12 showing an apparatus 500 of the present invention having a series of four motors 502 aligned with a common axis 504. The number of motors can be any suitable number. Each of the motors 502 has an outer stationary stator 518, an inner rotatable rotor 516, and a hollow shaft 512 rotatable with the inner rotor 516. The hollow shaft 512 can be positioned on bearings 514 to enable the hollow shaft 512 to rotate in relation to the stationary central shaft 510. The bearings 514 can be any suitable bearing. In order to maintain the stator 518 stationary, the stator 518 can be connected to the stationary central shaft 512 via a bracket 520. The motors 502 can be purchased from Kollmorgen Inland Motor of Radford, Virginia.

Each of the hollow rotatable shafts 512 is linked to a transferring device 522 via a rigid transmitting mechanism 524 to enable the transferring device 522 to rotate together with the hollow rotatable shaft 512 around the common axis 504. The rigid transmitting mechanism 524 can be attached to the hollow rotatable shaft 512 and the transferring device 522 by any suitable means. The transferring device 522 can be supported by a bearing 526 which can be supported by the stationary central shaft 510. The bearing 526 can be any suitable bearing. Alternatively, if desired, the transferring device 522 can be attached to a second motor 502 to provide increased driving capability and/or structural support for the transferring device. Further, alternatively, the transferring device 522 can be driven by any desirable number of motors.

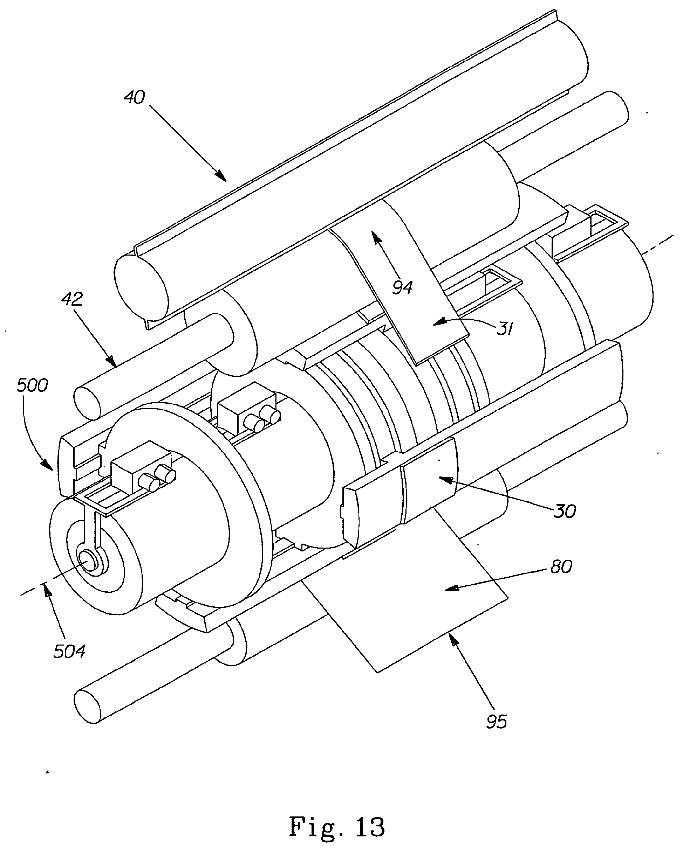

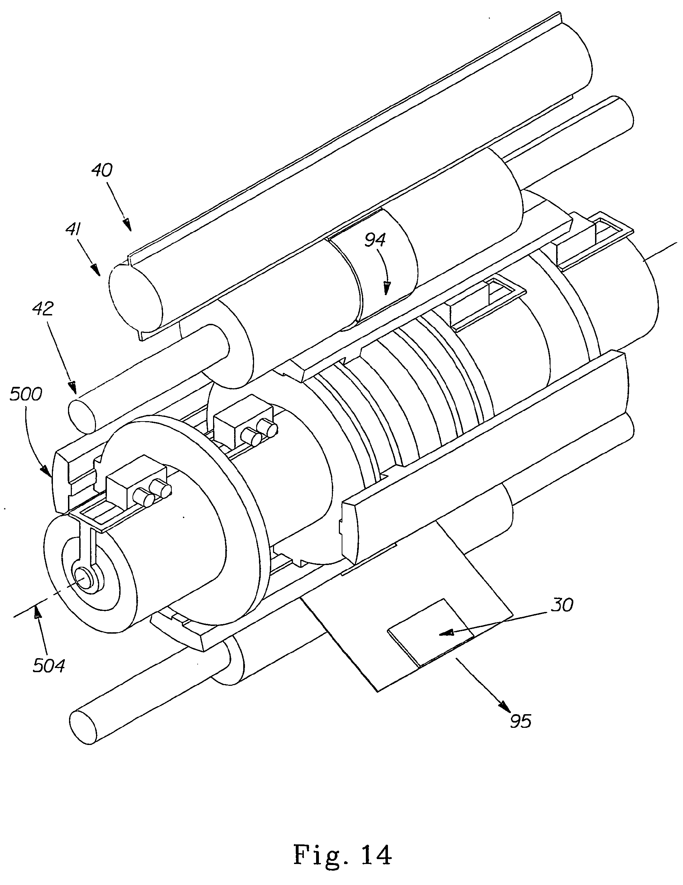

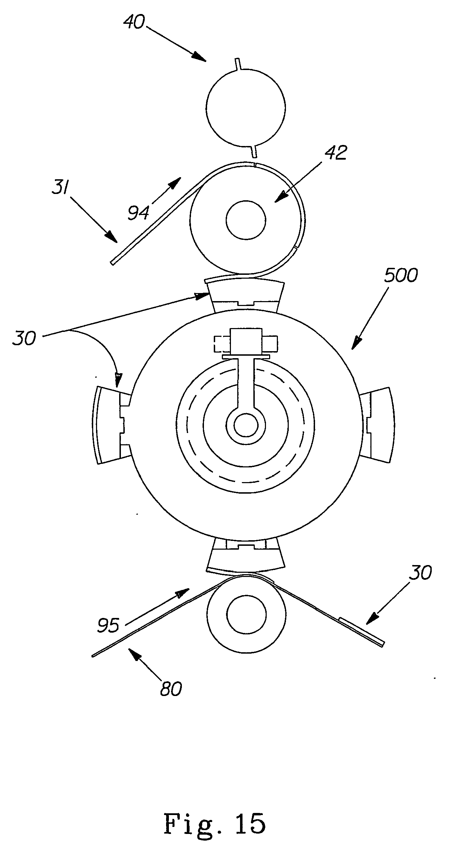

Figures 13-15 illustrate an example of how the apparatus 500 can be used in combination with the cutting device 40 (previously shown in Figure 4), similarly in all or any aspects to the cutting application described in detail hereinabove, including the alternative omission of the anvil roll 42 and the alternative severing of the material web 31 by a speed differential between adjacent transferring devices.

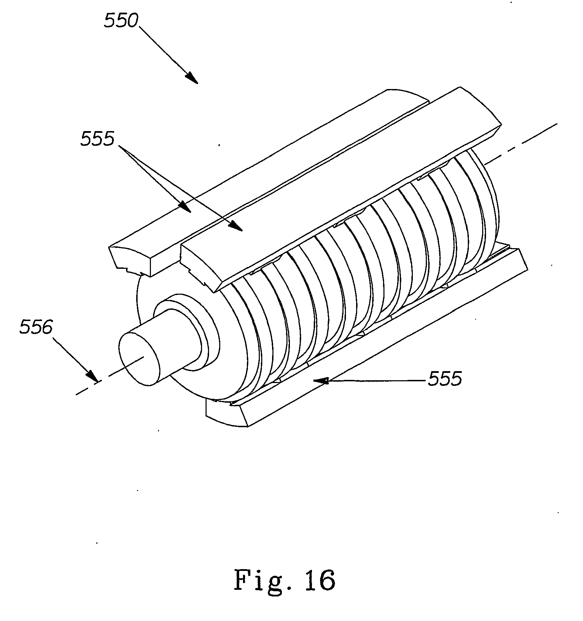

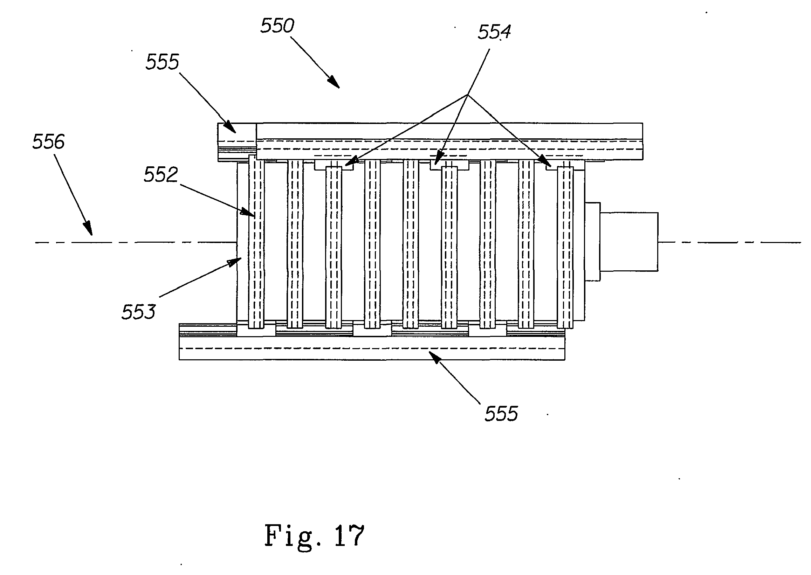

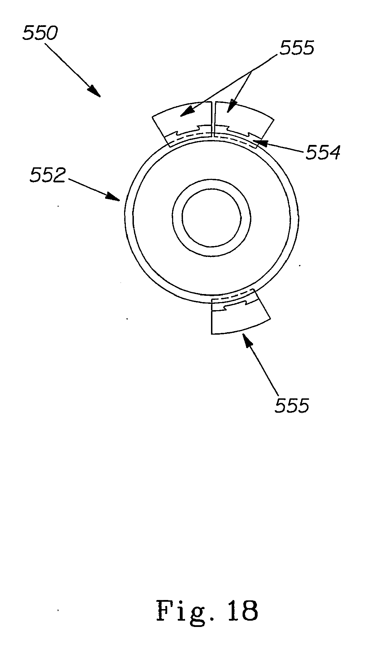

Figures 16-18 illustrate another embodiment of an apparatus 550 of the present invention forming a series of nine motors 551 aligned in relation to the central common axis 556. The motor 551 is generally known as a linear motor having a stationary track 552 forming a path 553 and at least one rider 554 movable on the stationary track 552 on the path 553. The motor 551 can be any suitable linear motor, including linear motors available from Anorad Corporation of New York. For example, the stationary track 552 can include armature windings and/or magnetic materials. The rider 554 can be a permanent magnet, coils, or combinations thereof. The rider is attached to the transferring device 555 for moving the transferring device 555 around the central common axis 556. The number of riders attached to the transferring device can vary from a single rider 554 to a multiplicity of riders 554, either controlled independently or jointly. As shown in Figures 16-18, each transferring device 555 is moved by three riders 554.

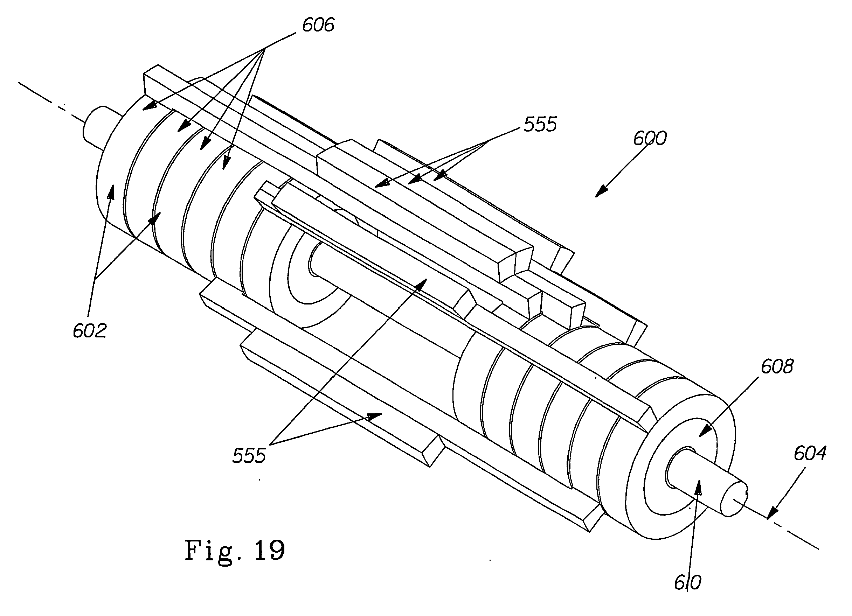

Figure 19 illustrates another embodiment of an apparatus 600 of the present invention forming a series of twelve motors 602 aligned in relation to a central common axis 604. The motor 602 can be any conventional motor having a rotatable outer rotor 606 and a stationary inner stator 608. As shown, each stator 608 is attached to a stationary central shaft 610, however, the motors 602 can be aligned without the use of the central shaft 610, for example, by connecting the motors 602 sideways to each other, directly or indirectly, via inner stationary stators 608. Each of the outer rotors 606 can be directly or indirectly attached to one or more of the transferring devices 550 for moving the transferring device in a programmed motion.

Alternatively, the apparatus of the present invention can include other types of programmable electrical motors suitable to provide a rotational motion of the outer component of the motor. For example, the motors wherein the stator and rotor are disposed parallel to each other as opposed to the concentric arrangements of the motors described therein above.

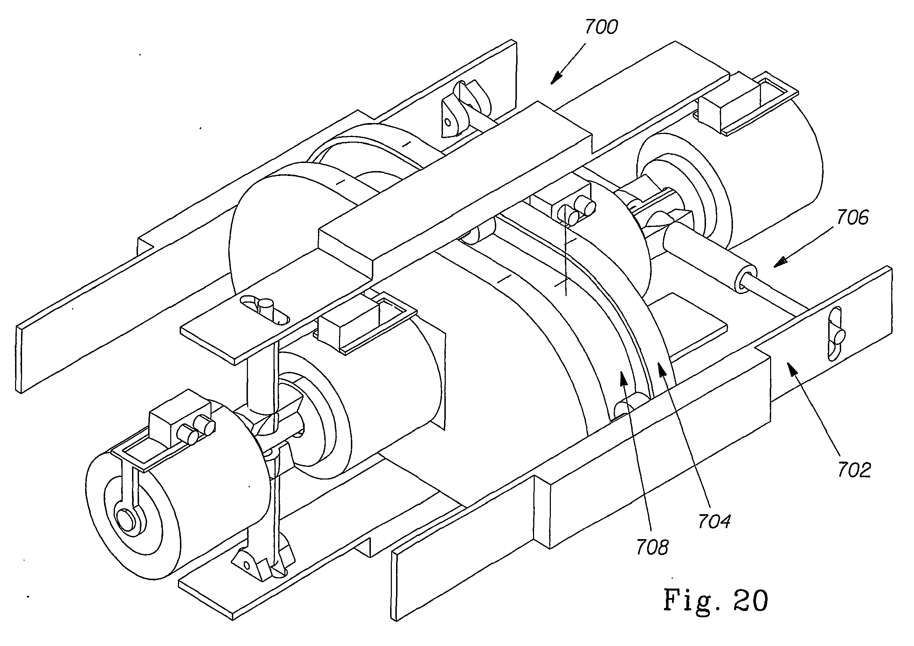

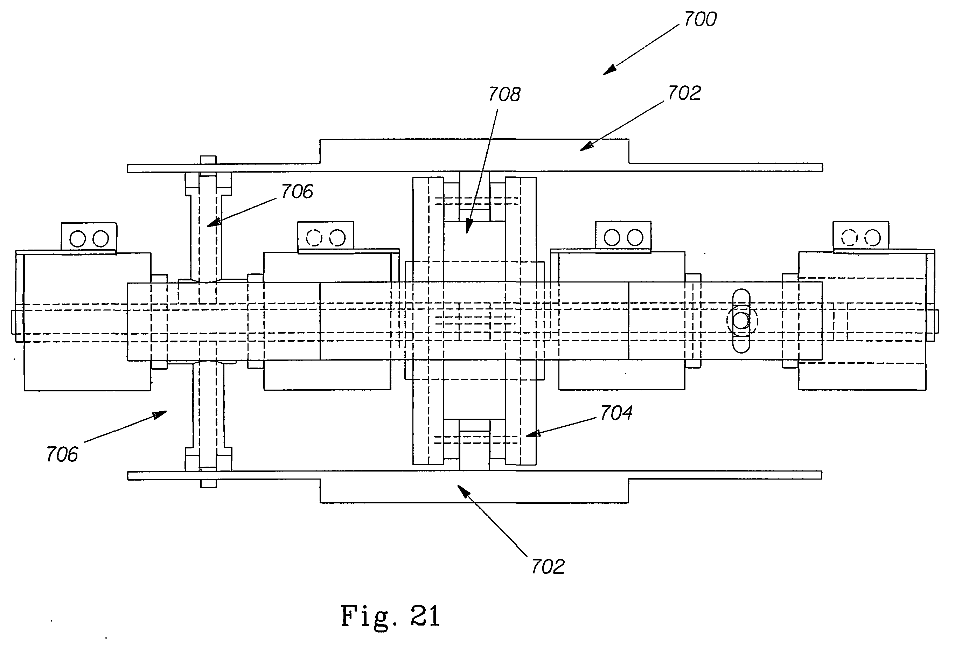

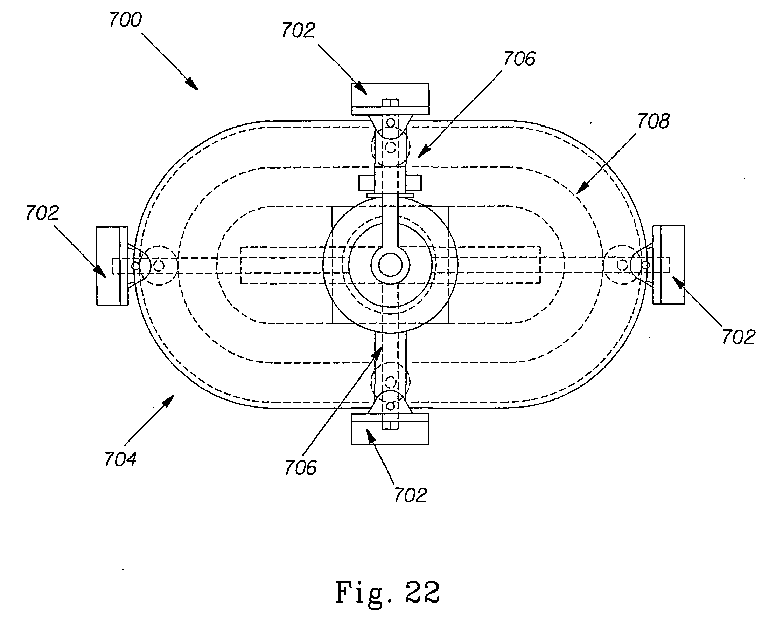

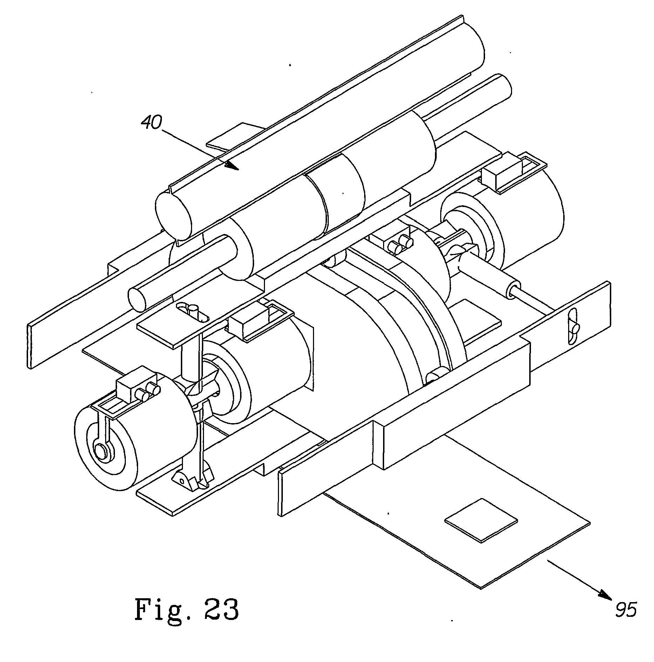

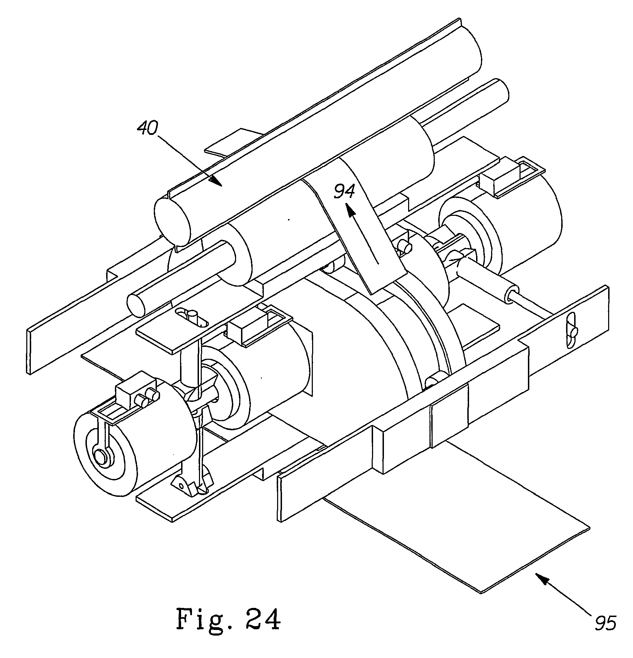

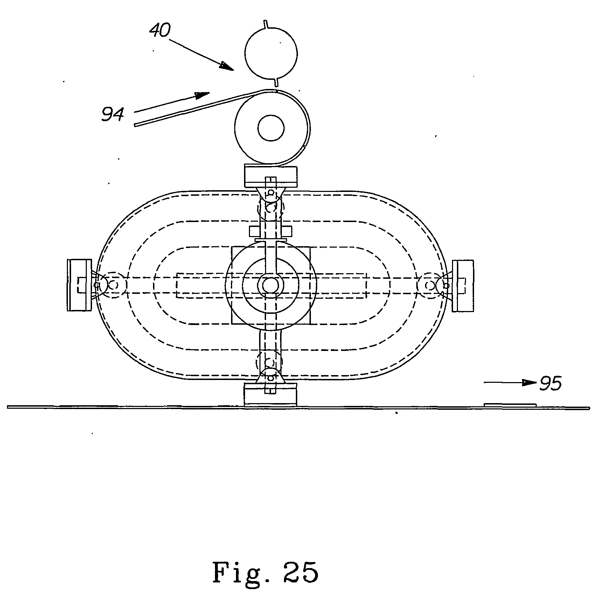

Figures 20-22 illustrate another embodiment of an apparatus 700 wherein the transferring devices 702 are movable in an orbital path 704 rather than a circular path as described in the apparatus 500 of Figures 10-12. The orbital path 704 of the transferring devices 702 is provided via extensible connections 706 in combination with a certain cam profile 708 of the orbital path 704. The motors of apparatus 700 can be any suitable motor described above. Figures 23-25 illustrate the above embodiment including the cutting device 40 as previously shown and described above.

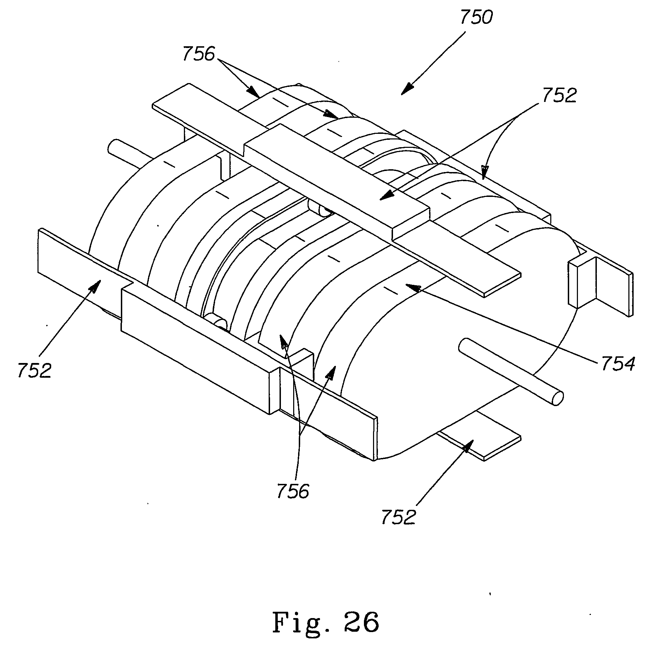

Figure 26 illustrates another embodiment of an apparatus 750 wherein the transferring devices 752 are movable in an orbital path 754 provided by the shape of the stationary track 756 of the programmable linear motor.

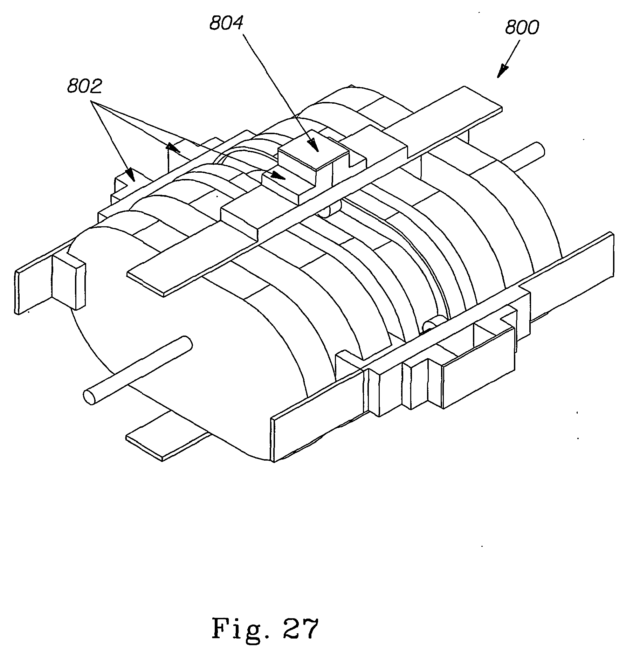

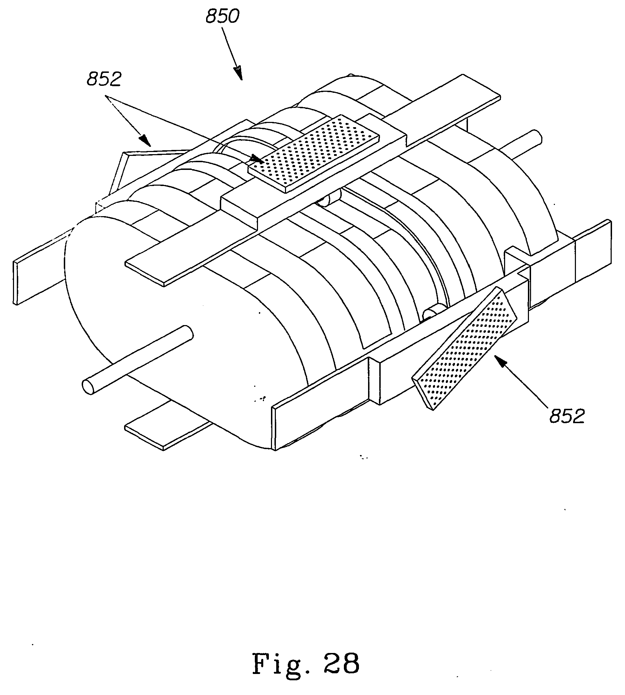

Other alternative additional processes can include operations associated with modifying the physical dimensions, the material properties, and/or the orientation of the discrete parts in relation to the substrate to which it will be attached. Specifically, such operations can include stretching, activation, rotation and the like by any means known to those skilled in the art. For example, Figure 27 illustrates another embodiment of the apparatus 800 of the present invention including a stretching device 802 for stretching the discrete part 804 before transferring said parts to the substrate. Further, Figure 28 illustrates another embodiment of the apparatus 850 of the present invention including a rotating device 852 for rotating discrete parts before transferring said parts to the substrate.

Further, certain applications can benefit from having a flexible pitch capability. Typical examples of pitched systems include knife rolls, printing rolls, bonding rolls and the like, as well as the discrete parts transferring applications described thereinabove. The pitch of a system such as a knife roll can be typically changed by adding additional tooling on a roll (e.g., the number of knives on a roll determines the number of pitches per a revolution of a roll), however such approach is not convenient for a rapid change. Other alternatives can include varying the rotational speed of the roll, adding or subtracting spacer plates underneath the tooling to change radial distance from the axis of rotation, or operating the tooling at the appropriate cycle rate but a different surface speed from the substrate it is interacting with. The embodiments of the apparatus of the present invention described hereinabove can offer the benefit of matched speed interactions with the substrate at a desired cycle rate and without a need for physically modifying the system when changing from pitch length to pitch length, even if the pitch length varies either intentionally or unintentionally from product to product during continuous operation.

**Claims**

1.  An apparatus (20) for receiving parts (30) traveling at a first speed through a receiving zone and applying the parts to a carrier (80) traveling at a second speed through an application zone (23), the apparatus comprising:

    at least two independent programmable motors (64A, 64B) , and
    at least two transferring devices (50A, 50B) for receiving the parts in the receiving zone (21) and applying the parts in the application zone, at least one of the transferring devices being coupled to each of the programmable motors for moving the transferring devices in an orbital path, **characterized** that the programmable motors (64A, 64B) and the transferring devices (50A, 50B) are aligned in relation to a common axis, and

wherein the programmable motors maintain the transferring devices at first surface speeds in the receiving zone (21) as the transferring devices pick up the parts (30) and maintain The transferring devices at second surface speeds in the application zone (23) as the transferring devices apply the parts to the carrier.

2. The apparatus (20) as defined in claim 1 wherein at least one of the programmable motors (64A, 64B) is selected from the group consisting of a motor having a hollow rotatable shaft, a linear motor having a stationary track rail, a motor having a rotatable outer rotor and a stationary inner stator, and a motor having a rotor rotatable around a stationary component of a motor.

3. The apparatus (20) as defined in claim 1 wherein the programmable motors (64A, 64B) are located on a stationary central shaft (512) coaxially with the common axis.

4. The apparatus (20) as defined in claim 2 wherein at least one of the programmable motors (64A, 64B) is coupled to at least one of the transferring devices (50A, 50B) via a transmitting mechanism (524) for moving the at least one of the transferring devices.

5. The apparatus (20) as defined in claim 4 wherein the transmitting mechanism (524) is a rigid connection or an extensible connection.

6. The apparatus (20) as defined in claim 2 wherein the stationary track rail (552) of the linear motor includes a plurality of armature windings therein or a magnetic material therein.

7. The apparatus (20) as defined in claim 6 wherein the linear motor includes at least one rider (554) movable on the stationary track rail (552).

8. The apparatus (20) as defined in any of the preceding claims wherein the first and second surface speeds of the transferring devices (50A, 50B) are substantially constant.

9. The apparatus (20) as defined in any of the preceding claims wherein the first and second surface speeds of the transferring devices (50A, 50B) are variable.

10. The apparatus (20) as defined in any of the preceding claims wherein either the first surface speeds of the transferring devices (50A, 50B)or the second surface speeds of the transferring devices are variable.

11. The apparatus (20) as defined in any of the preceding claims wherein the first surface speeds of the transferring devices (50A, 50B) are substantially equal to the first speed of the parts in the receiving zone (21) and the second surface speeds of the transferring devices are substantially equal to the second speed of the carrier (80) in the application zone (23).

12. The apparatus (20) as defined in any of the preceding claims wherein at least one of the transferring devices (50A, 50B) comprises at least one shell segment (31) having an outer surface, the shell segment is movable along the orbital path that passes through the receiving zone (21) and the application zone (23) during movement of the at least one of the transferring devices (50A, 50B), the shell segment collects at least one of the parts (30) in the receiving zone and holds the at least one of the parts against the outer surface utilizing a vacuum, electrostatics, or a coefficient of friction before applying the at least one of the parts to the carrier in the application zone.

13. The apparatus (20) as defined in claim 12 wherein the shell segment (51) utilizes a vacuum, electrostatics, or surface coefficient of friction to hold the parts (30) to the outer surface.

14. The apparatus (20) as defined in claim 12 wherein the outer surface of the shell segment (51) is an arcuate surface or a flat surface.

15. The apparatus (20) as defined in any of the preceding claims wherein the carrier (80) comprises a web substrate, a belt or a drum.

16. The apparatus (20) as defined in any of the preceding claims wherein the orbital path forms a circle or any trajectory including one or more curvilinear or rectilinear sections.

17. The apparatus (20) as defined in any of the preceding claims wherein at least one of the transferring devices (50A, 50B) is coupled to more than one of the programmable motors (64A, 64B).

18. The apparatus (20) as defined in any of the preceding claims further comprising an applicator for performing a secondary process on the parts between the receiving zone (21) and the application zone (23).

19. The apparatus (20) as defined in any of the preceding claims further comprising a cutting device (40) wherein a continuous web of material is cut into parts at the receiving zone (21).

**Patentansprüche**

1. Vorrichtung (20) zum Aufnehmen von Teilen (30), die sich bei einer ersten Geschwindigkeit durch einen Aufnahmebereich bewegen, und Auflegen der Teile auf einen Träger (80), der sich bei einer zweiten Geschwindigkeit durch einen Applikationsbereich (23) bewegt, wobei die Vorrichtung Folgendes umfasst:

   mindestens zwei unabhängige programmierbare Motoren (64A, 64B); und
   mindestens zwei Beförderungsvorrichtungen (50A, 50B) zum Aufnehmen der Teile in dem Aufnahmebereich (21) und Auflegen der Teile in dem Applikationsbereich, wobei mindestens eine der Beförderungsvorrichtungen mit jedem der programmierbaren Motoren zum Bewegen der Beförderungsvorrichtungen in einer Umlaufbahn verbunden ist,

   **dadurch gekennzeichnet, dass** die programmierbaren Motoren (64A, 64B) und die Beförderungsvorrichtungen (50A, 50B) in Bezug auf eine gemeinsame Achse ausgerichtet sind, und
   wobei die programmierbaren Motoren die Beförderungsvorrichtungen bei ersten Oberflächengeschwindigkeiten in dem Aufnahmebereich (21) halten, während die Beförderungsvorrichtungen die Teile (30) aufnehmen, und die Beförderungsvorrichtungen bei zweiten Oberflächengeschwindigkeiten in dem Applikationsbereich (23) halten, während die Beförderungsvorrichtungen die Teile auf den Träger auflegen.

2. Vorrichtung (20) nach Anspruch 1, wobei mindestens einer der programmierbaren Motoren (64A, 64B) ausgewählt ist aus der Gruppe, bestehend aus einem Motor mit einer drehbaren Hohlwelle, einem linearen Motor mit einer stationären Führungsschiene, einem Motor mit einem drehbaren äußeren Rotor und einem stationären inneren Stator und einem Motor, der um eine stationäre Komponente eines Motors gedreht werden kann.

3. Vorrichtung (20) nach Anspruch 1, wobei die programmierbaren Motoren (64A, 64B) auf einer stationären zentralen Welle (512) koaxial mit der gemeinsamen Achse angeordnet sind.

4. Vorrichtung (20) nach Anspruch 2, wobei mindestens einer der programmierbaren Motoren (64A, 64B) mit mindestens einer der Beförderungsvorrichtungen (50A, 50B) durch einen Übertragungsmechanismus (524) zum Bewegen der mindestens einen der Beförderungsvorrichtungen verbunden ist.

5. Vorrichtung (20) nach Anspruch 4, wobei der Übersetzungsmechanismus (524) eine starre Verbindung oder eine ausdehnbare Verbindung ist.

6. Vorrichtung (20) nach Anspruch 2, wobei die stationäre Führungsschiene (552) des linearen Motors mehrere Ankerwicklungen darin oder ein magnetisches Material darin aufweist.

7. Vorrichtung (20) nach Anspruch 6, wobei der lineare Motor mindestens einen Sitz (554) aufweist, der auf der stationären Führungsschiene (552) bewegt werden kann.

8. Vorrichtung (20) nach einem der vorstehenden Ansprüche, wobei die ersten und die zweiten Oberflächengeschwindigkeiten der Beförderungsvorrichtungen (50A, 50B) im Wesentlichen konstant sind.

9. Vorrichtung (20) nach einem der vorstehenden Ansprüche, wobei die ersten und die zweiten Oberflächengeschwindigkeiten der Beförderungsvorrichtungen (50A, 50B) variabel sind.

10. Vorrichtung (20) nach einem der vorstehenden Ansprüche, wobei entweder die ersten Oberflächengeschwindigkeiten der Beförderungsvorrichtungen (50A, 50B) oder die zweiten Oberflächengeschwindigkeiten der Beförderungsvorrichtungen variabel sind.

**11.** Vorrichtung (20) nach einem der vorstehenden Ansprüche, wobei die ersten Oberflächengeschwindigkeiten der Beförderungsvorrichtungen (50A, 50B) im Wesentlichen der ersten Geschwindigkeit der Teile in dem Aufnahmebereich (21) entsprechen und die zweiten Oberflächengeschwindigkeiten der Beförderungsvorrichtungen im Wesentlichen der zweiten Geschwindigkeit des Trägers (80) in dem Applikationsbereich (23) entsprechen.

**12.** Vorrichtung (20) nach einem der vorstehenden Ansprüche, wobei mindestens eine der Beförderungsvorrichtungen (50A, 50B) mindestens ein Schalensegment (31) mit einer äußeren Oberfläche umfasst, wobei das Schalensegment entlang der Umlaufbahn bewegt werden kann, die während der Bewegung der mindestens einen der Beförderungsvorrichtungen (50A, 50B) durch den Aufnahmebereich (21) und den Applikationsbereich (23) verläuft, wobei das Schalensegment mindestens eines der Teile (30) in dem Aufnahmebereich erfasst und das mindestens eine der Teile durch ein Vakuum, Elektrostatik oder einen Reibungskoeffizienten vor Auflegen des mindestens einen der Teile auf den Träger in dem Applikationsbereich gegen die äußere Oberfläche hält.

**13.** Vorrichtung (20) nach Anspruch 12, wobei das Schalensegment (51) ein Vakuum, Elektrostatik oder einen Oberflächenreibungskoeffizienten benutzt, um die Teile (30) an die äußere Oberfläche zu halten.

**14.** Vorrichtung (20) nach Anspruch 12, wobei die äußere Oberfläche des Schalensegments (51) eine bogenförmige Oberfläche oder eine flache Oberfläche ist.

**15.** Vorrichtung (20) nach einem der vorstehenden Ansprüche, wobei der Träger (80) ein Bahnsubstrat, ein Band oder eine Walze umfasst.

**16.** Vorrichtung (20) nach einem der vorstehenden Ansprüche, wobei die Umlaufbahn einen Kreis oder jeden beliebigen Bahnverlauf bildet, der einen oder mehrere krummlinige oder geradlinige Abschnitte einschließt.

**17.** Vorrichtung (20) nach einem der vorstehenden Ansprüche, wobei mindestens eine der Beförderungsvorrichtungen (50A, 50B) mit mehr als einem der programmierbaren Motoren (64A, 64B) verbunden ist.

**18.** Vorrichtung (20) nach einem der vorstehenden Ansprüche, ferner umfassend einen Applikator zum Ausführen eines sekundären Verfahrens an den Teilen zwischen dem Aufnahmebereich (21) und dem Applikationsbereich (23).

**19.** Vorrichtung (20) nach einem der vorstehenden Ansprüche, ferner umfassend eine Schneidvorrichtung (40), wobei eine kontinuierliche Materialbahn an dem Aufnahmebereich (21) in Teile geschnitten wird.

**Revendications**

**1.** Appareil (20) pour recevoir des pièces (30) se déplaçant à une première vitesse à travers une zone de réception et appliquer les pièces sur un transporteur (80) se déplaçant à une deuxième vitesse à travers une zone d'application (23), l'appareil comprenant :

au moins deux moteurs programmables indépendants (64A, 64B) ; et
au moins deux dispositifs de transfert (50A, 50B) pour recevoir les pièces dans la zone de réception (21) et appliquer les pièces dans la zone d'application, au moins un des dispositifs de transfert étant couplé à chacun des moteurs programmables pour déplacer les dispositifs de transfert dans une trajectoire orbitale,

**caractérisé en ce que** les moteurs programmables (64A, 64B) et les dispositifs de transfert (50A, 50B) sont alignés en relation avec un axe commun, et
dans lequel les moteurs programmables maintiennent les dispositifs de transfert à des premières vitesses de surface dans la zone de réception (21) à mesure que les dispositifs de transfert prennent les pièces (30) et maintiennent les dispositifs de transfert à des deuxièmes vitesses de surface dans la zone d'application (23) à mesure que les dispositifs de transfert appliquent les pièces sur le transporteur.

**2.** Appareil (20) tel que défini dans la revendication 1, dans lequel au moins un des moteurs programmables (64A, 64B) est choisi dans le groupe constitué d'un moteur ayant un arbre pivotant creux, un moteur linéaire ayant un rail de piste stationnaire, un moteur ayant un rotor externe pivotant et un stator interne stationnaire, et un moteur ayant un rotor pivotant autour d'un composant stationnaire d'un moteur.

3. Appareil (20) tel que défini dans la revendication 1, dans lequel les moteurs programmables (64A, 64B) sont situés sur un arbre central stationnaire (512) coaxialement avec l'axe commun.

4. Appareil (20) tel que défini dans la revendication 2, dans lequel au moins un des moteurs programmables (64A, 64B) est couplé à au moins un des dispositifs de transfert (50A, 50B) via un mécanisme de transmission (524) pour déplacer au moins un des dispositifs de transfert.

5. Appareil (20) tel que défini dans la revendication 4, dans lequel le mécanisme de transmission (524) est un raccordement rigide ou un raccordement extensible.

6. Appareil (20) tel que défini dans la revendication 2, dans lequel le rail de piste stationnaire (552) du moteur linéaire inclut une pluralité d'enroulements d'induit dedans ou un matériau magnétique dedans.

7. Appareil (20) tel que défini dans la revendication 6, dans lequel le moteur linéaire inclut au moins un cavalier (554) mobile sur le rail de piste stationnaire (552).

8. Appareil (20) tel que défini dans l'une quelconque des revendications précédentes, dans lequel les premières et deuxièmes vitesses de surface des dispositifs de transfert (50A, 50B) sont essentiellement constantes.

9. Appareil (20) tel que défini dans l'une quelconque des revendications précédentes, dans lequel les premières et deuxièmes vitesses de surface des dispositifs de transfert (50A, 50B) sont variables.

10. Appareil (20) tel que défini dans l'une quelconque des revendications précédentes, dans lequel ou les premières vitesses de surface des dispositifs de transfert (50A, 50B) ou les deuxièmes vitesses de surface des dispositifs de transfert sont variables.

11. Appareil (20) tel que défini dans l'une quelconque des revendications précédentes, dans lequel les premières vitesses de surface des dispositifs de transfert (50A, 50B) sont essentiellement égales à la première vitesse des pièces dans la zone de réception (21) et les deuxièmes vitesses de surface des dispositifs de transfert sont essentiellement égales à la deuxième vitesse du transporteur (80) dans la zone d'application (23).

12. Appareil (20) tel que défini dans l'une quelconque des revendications précédentes, dans lequel au moins un des dispositifs de transfert (50A, 50B) comprend au moins un segment de coque (31) ayant une surface externe, le segment de coque est mobile le long de la trajectoire orbitale qui passe à travers la zone de réception (21) et la zone d'application (23) durant le mouvement d'au moins un des dispositifs de transfert (50A, 50B), le segment de coque collecte au moins une des pièces (30) dans la zone de réception et maintient l'au moins une des pièces contre la surface externe en utilisant un vide, une propriété électrostatique, ou un coefficient de frottement avant application de l'au moins une des pièces sur le transporteur dans la zone d'application.

13. Appareil (20) tel que défini dans la revendication 12, dans lequel le segment de coque (51) utilise un vide, une propriété électrostatique, ou un coefficient de frottement de surface pour maintenir les pièces (30) sur la surface externe.

14. Appareil (20) tel que défini dans la revendication 12, dans lequel la surface externe du segment de coque (51) est une surface arquée ou une surface plate.

15. Appareil (20) tel que défini dans l'une quelconque des revendications précédentes, dans lequel le transporteur (80) comprend un substrat de nappe, une courroie ou un tambour.

16. Appareil (20) tel que défini dans l'une quelconque des revendications précédentes, dans lequel la trajectoire orbitale forme un cercle ou n'importe quelle trajectoire incluant une ou plusieurs sections curvilignes ou rectilignes.

17. Appareil (20) tel que défini dans l'une quelconque des revendications précédentes, dans lequel au moins un des dispositifs de transfert (50A, 50B) est couplé à plus d'un des moteurs programmables (64A, 64B).

18. Appareil (20) tel que défini dans l'une quelconque des revendications précédentes, comprenant, en outre, un applicateur pour exécuter un processus secondaire sur les pièces entre la zone de réception (21) et la zone d'application (23).

19. Appareil (20) tel que défini dans l'une quelconque des revendications précédentes, comprenant, en outre, un dispositif de coupe (40) dans lequel une nappe continue de matériau est coupée en pièces à la zone de réception (21).

Fig. 1

Fig. 2

Fig. 3

Fig. 4

Fig. 5

W

0%   10%   20%   30%   40%   50%   60%   70%   80%   90%   100%

21

23

Fig. 6

W

0%   10%   20%   30%   40%   50%   60%   70%   80%   90%   100%

21

23

Fig. 7

EP 1 303 240 B1

Fig. 8a

Fig. 8b

23

W

0%  10%  20%  30%  40%  50%  60%  70%  80%  90%  100%

21

23

Fig. 8c

251A

250

300

151A

272

151C

251B

172

262

264

151B

150

251C

164

162

Fig. 9a

Fig. 9b

Fig. 10

Fig. 11

Fig. 12

EP 1 303 240 B1

Fig. 13

Fig. 14

Fig. 15

Fig. 16

Fig. 17

550

555

554

552

555

Fig. 18

Fig. 19

Fig. 20

EP 1 303 240 B1

Fig. 21

EP 1 303 240 B1

Fig. 22

Fig. 23

EP 1 303 240 B1

40

94

95

Fig. 24

Fig. 25

Fig. 26

Fig. 27

850

852

852

Fig. 28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5693195 A, Schmitz
- US 6022443 A, Rajala and Makovec
- US 5766406 A, Bahn